# EUROPÄISCHE PATENTSCHRIFT

(11) **EP 3 629 940 B1**
(45) Veröffentlichungstag und Bekanntmachung des Hinweises auf die Patenterteilung: **03.05.2023**
(21) Anmeldenummer: 18732631.9
(22) Anmeldetag: 30.05.2018
(51) Int. Cl.: A61B 10/00

(54) **VORRICHTUNG ZUR ENTNAHME EINER FLÜSSIGKEITSPROBE**
DEVICE FOR COLLECTING A FLUID SAMPLE
APPAREIL POUR PRÉLEVER UN ÉCHANILLION DE FLUIDE

(30) Priorität: 02.06.2017 AT 504682017
(43) Veröffentlichungstag der Anmeldung: 08.04.2020
(73) Patentinhaber: Swacrit Systems GmbH, 6060 Hall in Tirol (AT)
(72) Erfinder: TSCHIGG, Andreas, 6071 Aldrans (AT)
(74) Vertreter: Torggler & Hofmann Patentanwälte - Innsbruck
(86) Internationale Anmeldenummer: PCT/AT2018/060113
(87) Internationale Veröffentlichungsnummer: WO 2018/218269

(56) Entgegenhaltungen:
- FR-A1- 2 331 318
- US-A- 4 637 403
- US-B1- 6 485 439

## Beschreibung

Die Erfindung betrifft eine Vorrichtung zur Entnahme einer Flüssigkeitsprobe mit den Merkmalen des Oberbegriffs des Anspruchs 1.

Derartige Vorrichtungen zählen bereits zum Stand der Technik und werden beispielsweise in der US 5,520,650 A, der DE 69919690 T2, der DE 2543576 A1 und der DE 69130926 T2 gezeigt.

Weiters sind aus der FR 2331318 A1 und der US 4,637,403 A Vorrichtungen zur Entnahme von Blut bekannt.

Diese Vorrichtungen dienen zur Entnahme von Flüssigkeitsproben oder zum Setzen von Kathedern, Sonden oder Kanülen an einem Körper eines Lebewesens, beispielsweise eines Menschen oder eines Tieres. Die Flüssigkeitsprobe oder die Flüssigkeit werden dabei aus einem Hohlraum im Inneren des Körpers entnommen. Ein solcher Hohlraum kann beispielsweise die Harnblase eines Menschen sein. Hierbei spricht man insbesondere von einer suprapubischen Blasenpunktion, bei der der Urin direkt aus der Blase entnommen wird. Suprapubische Blasenpunktionen werden bei der Untersuchung des Harns durchgeführt, bei der eine Kontaminierung des Harnes oder der Harnprobe ausgeschlossen werden kann.

Beim normalen Ablauf des Harns durch die Harnröhre in ein Probegefäß kann es sein, dass die Probe durch den Ablauf des Harns durch die Harnröhre, durch das Umfeld außerhalb der Harnröhre bzw. durch das Probengefäß verunreinigt wird und diese Probe somit falsche Werte abliefern kann. Bei direkten Entnahme der Flüssigkeit aus dem Hohlraum des Körpers, wie beispielsweise bei der suprapubischen Blasenpunktion, wird dies verhindert. Problematisch dabei ist jedoch der eigentliche Eingriff zur Entnahme der Flüssigkeit aus dem Hohlraum des Körpers. Dieser ist oftmals mit Schmerzen verbunden und zeigt sich zudem als kompliziert für den Benutzer, der die Vorrichtung einsetzt. Der Vorgang an sich sollte so schnell wie möglich vonstattengehen, um den Eingriff so kurz und schmerzlos wie möglich durchzuführen. Bei den bekannten Vorrichtungen werden mehrere Arbeitsschritte benötigt, um die Entnahme der Flüssigkeitsprobe durchführen zu können. Weiters sind die Vorrichtungen oftmals sehr komplex ausgeführt, sodass der Benutzer bei der Probenentnahme mehrere Handgriffe durchführen muss, um die Probe entnehmen zu können.

Aufgabe der Erfindung ist es, die vorbeschriebenen Nachteile zu vermeiden und eine gegenüber dem Stand der Technik verbesserte Vorrichtung anzugeben.

Dies wird bei der erfindungsgemäßen Vorrichtung durch die Merkmale des Anspruchs 1 erreicht.

Dadurch, dass die Vorrichtung in einem Ladebetriebszustand durch den Auslöser in einen Vorschubsbetriebszustand bringbar ist, indem sich die Saugvorrichtung durch die Vorschubmechanik relativ zum Gehäuse bewegt, und anschließend selbsttätig in einen Saugbetriebszustand bringbar ist, indem automatisch das Volumen des Reservoirs durch eine Saugmechanik vergrößerbar ist, wird eine Vorrichtung geschaffen, die automatisch und selbsttätig alle für eine Probenentnahme benötigten Schritte durchführt. Die Vorrichtung wird im Ladebetriebszustand am Körper des Patienten angesetzt, der Auslöser wird betätigt und der Vorgang der Entnahme der Flüssigkeitsprobe wird automatisch und selbsttätig durchgeführt. Dabei wechselt die Vorrichtung automatisch vom Ladebetriebszustand in den Vorschubsbetriebszustand und daraufhin folgend in den Saugbetriebszustand. Es muss nicht jeder Zustand durch einen eigenen Handgriff betätigt oder ausgeführt werden.

Durch eine Auslösung erfolgt eine kurze und schnelle Entnahme der Flüssigkeitsprobe, was für den Patient als angenehmer empfunden wird als ein langfristiges Hantieren an einer Vorrichtung, die in oder an seinem Körper verweilt.

Wenn die Vorrichtung nach der Betätigung des Auslösers automatisch vom Ladebetriebszustand in den Vorschubsbetriebszustand und darauf folgend automatisch in den Saugbetriebszustand durch zumindest einen in oder an der Vorrichtung angeordneten Kraftspeicher - vorzugsweise in Form einer Feder - wechselbar ist, so entsteht ein schneller Wechsel der Betriebszustände und ein rascher Ablauf beim Entnehmen der Flüssigkeitsprobe. Die Kraftspeicher sind dabei zum Beispiel in Form von Druck- oder Zugfedern ausgebildet. Diese müssen vor der Verwendung der Vorrichtung in den Ladebetriebszustand gebracht werden. Dies kann beispielsweise durch den Benutzer selbst erfolgen oder die Vorrichtung ist bereits in diesem Ladebetriebszustand gebracht worden, sobald der Benutzer diese verwenden muss. So kann die Vorrichtung beispielsweise in einem Ladebetriebszustand verpackt sein, um steril und fertig für den Gebrauch beim Benutzer zur Verfügung zu stehen.

Wenn die Vorschubmechanik einen Vorschubkraftspeicher aufweist, welcher beim Entspannen die Vorschubmechanik im Vorschubsbetriebszustand in Bewegung setzt und die Saugmechanik einen Saugkraftspeicher aufweist, welcher beim Entspannen die Saugmechanik im Saugbetriebszustand in Bewegung setzt, so sind unterschiedliche Mechaniken und unterschiedliche, dafür vorgesehene, Kraftspeicher vorhanden, die getrennt voneinander in Bewegung setzbar sind. Weiters kann über die Unterschiede der Kraftspeicher erzielt werden, dass zum Beispiel die Vorschubmechanik einen stärkeren oder schwächeren Vorschubkraftspeicher aufweist als der Saugkraftspeicher der Saugmechanik.

Weiters ist es möglich, dass über die Verwendung eines Vorschubkraftspeichers und eines davon getrennten Saugkraftspeichers die Bewegungen der beiden Kraftspeicher unabhängig voneinander und zeitversetzt erfolgen können.

Wenn die Saugvorrichtung durch eine Injektionsspritze, vorzugsweise Einwegspritze, mit einer daran anordenbaren Nadel ausgebildet ist, kann auf ein handelsübliches Produkt zurückgegriffen werden, welches in das Gehäuse der Vorrichtung eingesetzt wird. Injektionsspritzen sind günstig zu erwerben, dies resultiert in einer kostengünstigen Vorrichtung. Die Vorrichtung kann als Einwegprodukt verwendet werden und nach der Entnahme der Probe entsorgt werden. Es kann jedoch auch vorgesehen sein, dass die Saugvorrichtung wechselbar in der Vorrichtung angeordnet ist, um nach einem Auslösen der Vorrichtung und einer Probenentnahme eine Mehrfachverwendung durch das Austauschen der Saugvorrichtung bzw. der Nadel zu realisieren. Dieses Beispiel entspricht nicht der beanspruchten Erfindung.

Erfindungsgemäss ist jedoch die Ausgestaltung der Vorrichtung als Einwegprodukt anzusehen. Aufgrund der Gefahr von Kontamination oder Ansteckungsgefahr bei einer Mehrfachverwendung, sollte nach einer einmaligen Benützung die Vorrichtung entsorgt werden.

Wenn die Bewegung der Vorschubmechanik relativ zum Gehäuse durch eine Vorschubbegrenzung einstellbar ist, so kann die Eindringtiefe der Nadel in den Körper des Patienten eingestellt werden. Die zu durchstechende Schichtdicke des Körpers zum Eindringen in den dahinterliegenden Hohlraum oder die Wanddicke zwischen Hohlraum und Körperoberfläche ist nicht immer gleich dimensioniert. Mittels Ultraschallmessung oder einem anderen Verfahren kann die zu durchdringende Schichtdicke ermittelt und an der Vorrichtung eingestellt werden. Dies erfolgt über die Vorschubbegrenzung. So kann beispielsweise bei einer suprapubischen Blasenpunktion via Ultraschall im Vorhinein der Füllstand der Blase ermittelt werden, zudem die Schichtdicke zwischen der Harnblase und der Bauchwand. Nach Ermittlung dieser Daten kann die Vorschubmechanik entsprechend über die Vorschubbegrenzung eingestellt werden. Bei einer nicht einstellbaren Vorrichtung kann es sein, dass die Nadel den Hohlraum komplett durchstößt, indem sich die für die Probe zu entnehmende Flüssigkeit befindet. Dabei können Verletzungen entstehen oder schwerwiegende gesundheitliche Probleme für den Patienten entstehen. Weiters muss bei einem Fehlversuch zum Einstechen in den Hohlraum zumindest ein weiterer Versuch durchgeführt werden, was zu zusätzlichem Unbehagen des Patienten führt.

Wenn, gemäß der Erfindung, eine Blockiervorrichtung vorgesehen ist, durch welche eine Mehrfachverwendung der Vorrichtung verhinderbar ist, so kann - wie bereits vorhin beschrieben - eine Mehrfachverwendung der Vorrichtung ausgeschlossen werden. Diese Blockiervorrichtung kann beispielsweise durch eine plastische oder auch elastische Deformierung im Inneren der Vorrichtung geschehen, wobei beispielsweise die Vorschubvorrichtung in ihrer Lage derart blockiert wird, dass ein erneutes Auslösen ausgeschlossen ist.

Als vorteilhaft hat es sich auch herausgestellt, dass das Gehäuse und die Saugvorrichtung zumindest abschnittsweise durch ein transparentes Material ausgebildet sind, wodurch der Füllstand des Reservoirs der Saugvorrichtung und der Betriebszustand der Vorrichtung erkennbar sind. Dies dient zur Kontrolle, ob die Entnahme der Flüssigkeitsprobe tatsächlich funktioniert hat, in welchem Betriebszustand sie sich befindet, bzw. auch zur Erkennung, ob die Vorrichtung bereits benutzt worden ist oder nicht.

Wenn eine Spannvorrichtung vorgesehen ist, durch welche ein Spannen des Vorschubkraftspeichers und/oder des Saugkraftspeichers und somit das Schaffen des Ladebetriebszustandes durchführbar ist. So kann über diese Spannvorrichtung die Vorrichtung in Betrieb gesetzt werden und der Ladebetriebszustand erreicht werden. Es kann jedoch auch sein, wie bereits oben beschrieben, dass die Vorrichtung bereits im geladenen Zustand beim Benützer ankommt.

Als vorteilhaft hat es sich dabei herausgestellt, dass die Spannvorrichtung auch zum Herausdrücken der Flüssigkeitsprobe aus dem Reservoir der Saugvorrichtung verwendbar ist. So besteht eine Art Doppelverwendung der Spannvorrichtung - einmal kann über diese die Vorrichtung geladen werden und in den Ladebetriebszustand gebracht werden und zusätzlich kann die Spannvorrichtung dazu verwendet werden, um die Flüssigkeitsprobe aus der Vorrichtung herauszudrücken. Beispielsweise kann die Spannvorrichtung an der Vorderseite (im Bereich der Nadel) eingesetzt werden, um die Vorrichtung zu spannen. Nach der vollzogenen Probenentnahme wird die Spannvorrichtung an der Kolbenbodenseite der Saugvorrichtung angesetzt, um das Reservoir entleeren zu können.

Wenn im Ladebetriebszustand der Vorschubkraftspeicher gespannt wird und der Saugkraftspeicher entladen, wobei sich bei einer Entladung des Vorschubkraftspeichers im Vorschubsbetriebszustand der Saugkraftspeicher aufgrund einer Kupplung zwischen der Vorschubmechanik und der Saugmechanik in einen gespannten Zustand bewegt und anschließend selbsttätig im Saugbetriebszustand in den entspannten Zustanden wechselbar ist, wobei das Volumen des Reservoirs an der Saugvorrichtung vergrößerbar ist, entstehen die nacheinander gereihten Bewegungszustände oder Betriebszustände der Vorrichtung aufgrund der Kupplung zwischen der Vorschubmechanik und der Saugmechanik. Diese Kupplung agiert dabei vollkommen selbstständig nach dem Betätigen des Auslösers. Es ist nicht notwendig, zuerst die Vorschubmechanik in Betrieb zu setzen und anschließend aktiv die Saugmechanik zu betätigen. Beide Mechaniken bewegen sich automatisch nacheinander nach Betätigung des Auslösers.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen
- Fig. 1: schematischer Ablauf einer Probenentnahme,
- Fig. 2: detaillierter schematischer Aufbau der Vorrichtung während einer Probenentnahme,
- Fig. 3: Verwendung der Spannvorrichtung und Inbetriebnahme der Vorrichtung zur Probenentnahme,
- Fig. 4: Vorrichtung im Ladebetriebszustand,
- Fig. 5: Vorrichtung im Vorschubsbetriebszustand,
- Fig. 6: Vorrichtung im Saugbetriebszustand,
- Fig. 7: Detail Ladebetriebszustand,
- Fig. 8: Detail Vorschubsbetriebszustand,
- Fig. 9: Detail Saugbetriebszustand und
- Fig. 10: Entleeren der Vorrichtung.

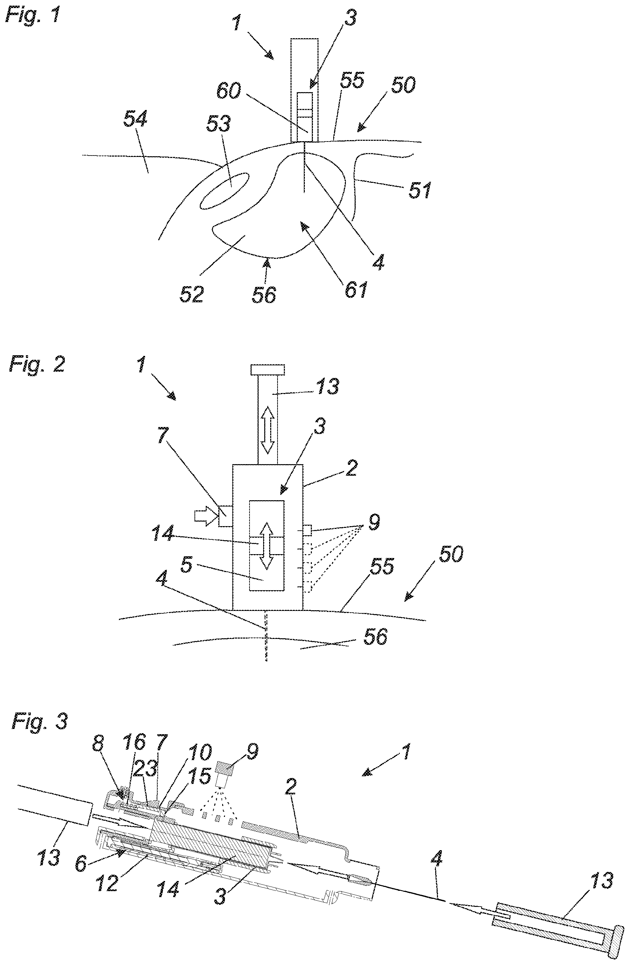

Fig. 1 zeigt die Vorrichtung 1 mit der darin befindlichen Saugvorrichtung 3, welche an einem Körper 50 angeordnet ist und mittels einer Nadel 4 in einen im Körper 50 befindlichen Hohlraum 56 eindringt. Als Beispiel wird in der Figur 1 eine suprapubische Blasenpunktion gezeigt. Dabei wird im Bereich oberhalb des Schambeines 53 die Vorrichtung 1 angesetzt und ausgelöst. So bewegt sich die Nadel 4 schlagartig durch die Bauchwand 55 und dringt in die Harnblase 52 ein. Nach dem Eindringen der Nadel 4 in die Harnblase 52 oder den Hohlraum 56 wird automatisch die Saugvorrichtung 3 aktiviert und aus der Flüssigkeit 61 die Flüssigkeitsprobe 60 durch die Saugvorrichtung 3 aufgenommen. Das Eindringen der Nadel 4 sollte im Bereich zwischen dem Schambein 53 und dem Bauchfell 51 stattfinden. Die exakte Position zum Ansetzen der Vorrichtung 1 wird im Vorfeld durch eine Untersuchung ermittelt. Die Fig. 2 zeigt schematisch, wie die Vorrichtung 1 an der Bauchwand 55 angesetzt wurde und in Betrieb gesetzt worden ist. Über die Spannvorrichtung 13 wurde die Vorrichtung in den Ladebetriebszustand LB gebracht. Die Spannvorrichtung 13 kann dabei ein fester Bestandteil der Vorrichtung 1 sein, oder auch separater Bestandteil, der mit der Vorrichtung 1 mitgeliefert worden ist. Im Falle der Figur 2 wird die Spannvorrichtung 13 als fester oder befestigbarer Bestandteil der Vorrichtung 1 gezeigt. Beispielsweise kann über ein Herausziehen oder Hineindrücken der Spannvorrichtung 13 ein Laden der Vorrichtung 1 erfolgen.

Die Spannvorrichtung 13 kann jedoch auch als separates Bauteil, beispielsweise vor dem Ansetzen an die Bauchdecke an der Vorderseite der Vorrichtung 1 angesetzt werden (im Bereich der Nadel 4) und dort entgegen der Vorschubrichtung der Saugvorrichtung eingedrückt werden. Nach dem Eindrücken und dem Bewegen der Vorrichtung 1 in den Ladebetriebszustand LB wird die Spannvorrichtung 13 entfernt.

Die Vorrichtung 1 wird im Ladebetriebszustand LB an der Bauchwand 55 angesetzt. Über den Auslöser 7 wird die Vorrichtung 1 vom Ladebetriebszustand LB in den Vorschubsbetriebszustand VB gebracht. Dabei durchstößt die Nadel 4 die Bauchwand 55 und dringt in den dahinterliegenden Hohlraum 56 ein. Die Eindringtiefe der Nadel 4 wurde im Vorfeld durch eine Untersuchung ermittelt und über die Vorschubbegrenzung 9 entsprechend eingestellt. Dies verdeutlichen die unterschiedlichen Steckplätze am Gehäuse 2 für die Vorschubbegrenzung 9. Nach dem Eindringen der Nadel 4 in den Hohlraum 56 geht die Vorrichtung 1 automatisch vom Vorschubsbetriebszustand VB in den Saugbetriebszustand SB über. Dies erfolgt selbsttätig und ohne eine weitere aktive Handlung des Benutzers. Es muss nur einmal der Auslöser 7 betätigt werden, um die unterschiedlichen Betriebszustände zu erreichen.

Im Saugbetriebszustand SB bewegt sich der Kolben 14 der Saugvorrichtung 3 und zieht somit die Flüssigkeit 61 aus dem Hohlraum 56 des Körpers 50. Nach der Durchführung der Probenentnahme wird die Vorrichtung 1 vom Körper 50 entfernt und die Probe wird über die Spannvorrichtung 13 oder eine andere Vorrichtung zum Ausdrücken der Flüssigkeitsprobe 60 aus dem Reservoir 5 gedrückt.

Fig. 3 zeigt die Vorbereitung der Vorrichtung 1 für die Probenentnahme. Dabei kann die Vorrichtung 1 über die Spannvorrichtung 13, welche nadelseitig in das Gehäuse 2 eingeschoben wird, in den Ladebetriebszustand LB gebracht werden. Die Nadel 4 kann bereits im Vorfeld schon an der Vorrichtung 1 befestigt werden oder auch zeitglich mittels Einführen der Spannvorrichtung 13. Die Spannvorrichtung 13 weist dabei einen Hohlraum auf, der die Nadel 4 umgibt. Es muss gewährleistet sein, dass die Nadel 4 nicht während dem Laden der Vorrichtung 1 kontaminiert wird. Die Nadel 4 kann auch ein fixer Bestandteil der Saugvorrichtung 3 sein. Die Nadel kann auch erst nach dem Laden durch die Spannvorrichtung 13 an der Saugvorrichtung 3 befestigt werden.

Nach der Ermittlung der Eindringtiefe für die Nadel 4 wird über die Vorschubbegrenzung 9 die Vorschubmechanik 6 in ihrer Bewegung relativ zum Gehäuse 2 eingeschränkt. In der Figur 3 wird gezeigt, wie die Vorschubbegrenzung über einen einsteckbaren Anschlag realisiert wird, der an einem Steckplatz im Gehäuse 2 einsteckbar ist. Es stehen mehrere Steckplätze zur Verfügung, welche in einem gewissen Abstand zueinander liegen. Der jeweilige Abstand zwischen den Steckplätzen, also die Rasterung, kann in einem Bereich zwischen 5 bis 15 mm liegen. Bevorzugt beträgt dieser eine Rasterung von 10 mm. Die Eindringtiefe, welche über die Vorschubbegrenzung einstellbar ist, kann somit beispielsweise von 20 mm bis 50 mm in 10 mm-Schritten eingestellt werden. Nach einer einmaligen Einstellung über die in der Figur 3 gezeigte Vorschubbegrenzung 9 kann diese nicht mehr gewaltlos aus dem Gehäuse 2 gezogen werden, da sie als Steckelement ausgebildet ist, welches formschlüssig in das Gehäuse 2 eingesteckt wird. Dies signalisiert zum einen, dass die Vorrichtung 1 geladen ist und nun betätigt werden kann, oder dass sie nach einer Betätigung bereits verwendet worden ist und nicht mehr wiederverwendet werden kann. Somit entsteht eine Mehrfachfunktion durch die Vorschubbegrenzung 9. Diese verhindert eine Mehrfachverwendung der Vorrichtung 1 und stellt gleichzeitig die maximale Eindringtiefe der Nadel 4 ein.

Das Aktivieren bzw. die Auslösung der Punktion und der Extraktion der Probe erfolgt über den Auslöser 7, nachdem der Benutzer das Gerät an der betreffenden Stelle am Körper positioniert hat. Zur Reduktion des Schmerzes kann eine Betäubungscreme oder eine andere lokale Narkose verwendet werden, welche vor der Punktion im Bereich der Punktion aufgetragen oder in einer anderen Form appliziert wird. Somit wird dem Patienten das Schmerzpotential des Eindringens der Nadel 4 genommen.

Nach dem Auslösen des Auslösers 7 wird der Vorschubmechanismus 6 in Betrieb gesetzt. Dies erfolgt durch den Vorschubkraftspeicher 12, der mit der Vorschubmechanik 6 gekoppelt ist. Dabei dringt die Nadel 4 in den Körper ein. In weiterer Folge wird automatisch über die Kupplung 23 die Saugmechanik 8 über den Saugkraftspeicher 16 in Bewegung gesetzt. Dabei füllt sich die Saugvorrichtung 3 mit der zu entnehmenden Flüssigkeitsprobe 60. Nach der Entnahme der Vorrichtung 1 kann die Flüssigkeitsprobe 60 aus der Saugvorrichtung 3 über ein Eindrücken des Kolbens 14 erfolgen. Das Eindrücken des Kolbens 14 kann direkt erfolgen oder auch über die Spannvorrichtung 13, welche im rückwärtigen Bereich der Vorrichtung 1 eingeschoben wird.

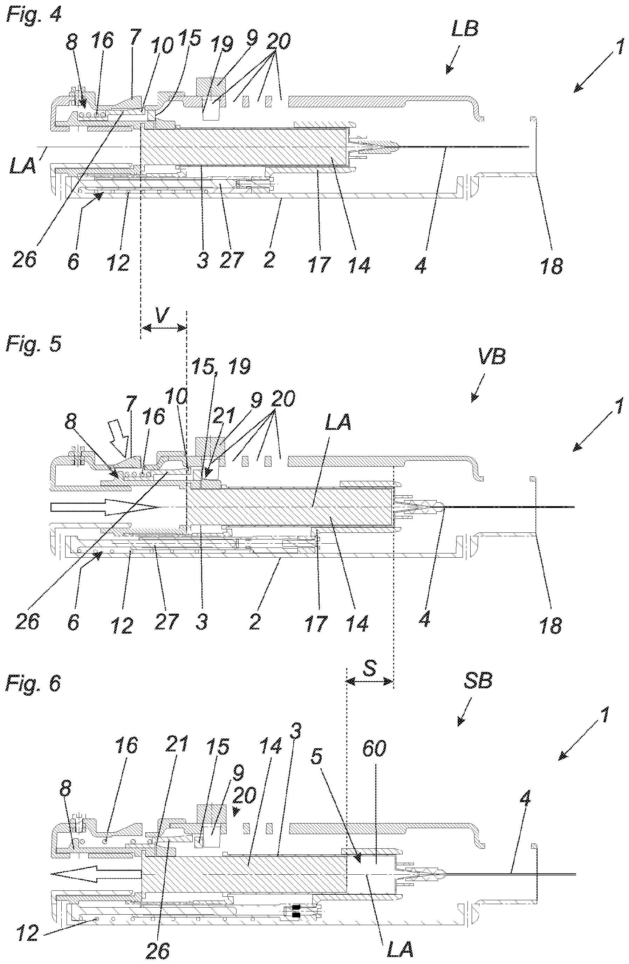

Fig. 4 zeigt die Vorrichtung 1 im Ladebetriebszustand LB. Die Nadel 4 ist bereits an der Saugvorrichtung 3 angeschlossen. Die Saugvorrichtung 3 befindet sich in einer Haltevorrichtung 17, welche relativ zum Gehäuse 2 der Vorrichtung 1 gelagert ist und mit der Vorschubmechanik 6 in Wirkverbindung steht. Bei der Bewegung der Vorschubmechanik 6 wird die Haltevorrichtung 17 relativ zum Gehäuse 2 bewegt, wodurch die Saugvorrichtung 3 mit der Nadel 4 im Inneren des Gehäuses 2 verschoben wird. Die Vorschubmechanik 6 wird ausgebildet durch eine zwischen dem Gehäuse 2 und der Haltevorrichtung 17 angeordnetes Führungselement 27. Entlang dem Führungselement 27 erstreckt sich der Vorschubkraftspeicher 12. Entlädt sich der Vorschubkraftspeicher 12, bewegt sich die Vorschubmechanik 6 entlang der Längsachse LA des Gehäuses 2 und entlang dem Führungselement 27.

Im Ladebetriebszustand LB ist der Vorschubkraftspeicher 12 geladen. Der Vorschubkraftspeicher 12 wird als Druckfeder ausgebildet. Durch ein Arretiermittel 10, welches mit einem Anschlagelement 26 in Wirkverbindung steht, wird der Vorschubkraftspeicher 12 an einer Entladung gehindert. In der Figur 4 wird dargestellt, wie sich das Arretiermittel 10 durch ein rückfederndes Element ausbildet und das Anschlagelement 26 durch ein Bestandteil des Gehäuses 2. Dieses rückfedernde Element ist ein Bestand der Kupplung 23. Wird nun der Auslöser 7 gedrückt, bewegt sich das rückfedernde Element quer zur Längsachse LA der Vorrichtung 1. Dabei gerät dieses außer Kontakt mit dem Anschlagelement 26 des Gehäuses 2. Daraufhin entlädt sich der Vorschubkraftspeicher 12 der Vorschubmechanik 6. Die gesamte Saugvorrichtung 3 bewegt sich entlang der Längsachse LA relativ zum Gehäuse 2. Dabei kann die Nadel 4 in den Körper des Patienten eindringen. Am vorderen Ende der Vorrichtung 1 bildet die Vorrichtung 1 einen Kontaktabschnitt 18 aus, durch welchen die Vorrichtung 1 mit dem Körper 50 in Verbindung tritt. Dies wird in der Fig. 5 näher gezeigt.

Die Vorschubbegrenzung 9 wurde bereits an einem der Steckplätze des Verstellabschnittes 20 am Gehäuse 2 eingesteckt und somit wurde die maximale Eindringtiefe der Nadel 4 definiert. Im Ladebetriebszustand LB befindet sich der Saugkraftspeicher 16 im geladenen Zustand. Durch ein Blockieren der Saugmechanik 8 im Ladebetriebszustand LB und im Vorschubbetriebszustand VB erfolgt keine Bewegung des Kolbens 14 der Saugvorrichtung 3.

Die Fig. 5 zeigt, wie der Auslöser 7 betätigt worden ist, dabei das rückfedernde Arretiermittel 10 außer Eingriff mit dem Gehäuse 2 oder dem Anschlagelement 26 gebracht worden ist. Die Vorschubmechanik 6 wird nun über den Vorschubkraftspeicher 12 angetrieben, die Saugvorrichtung 3 beschleunigt und stößt die Nadel 4 in den Körper des Patienten. Bei der Entspannung des Vorschubkraftspeichers 12 wird die Kupplung 23 mit dem Anschlag 15 gegen eine korrespondierende Anschlagsfläche 19 der Vorschubbegrenzung 9 bewegt. Durch den Kontakt des Anschlages 15 mit der korrespondierenden Anschlagsfläche 19 wird nun die Bewegung der Vorschubmechanik 6 gestoppt. Die maximale Eindringtiefe, welche vorher durch die Vorschubbegrenzung 9 eingestellt worden ist, ist erreicht. Sichtbar ist auch, wie das Rückhalteelement 21 sich unter die Vorschubbegrenzung 9 schiebt. Dadurch wird der nächste Betriebszustand der Vorrichtung 1 eingeleitet - der Saugbetriebszustande SB.

Fig. 6 zeigt wie sich der geladene Saugkraftspeicher 16 nun aufgrund der plastischen oder elastischen Verformung des Rückhaltelementes 21 in die entspannte Position bewegt. Das Rückhalteelement 21 ist als elastisch oder plastisch deformierbares Element ausgebildet, welches nach dem Kontakt mit der Vorschubbegrenzung deformiert ist und entlang der Längsachse LA verfahrbar an der Saugmechanik 8 angeordnet ist.

Die Deformation kann beispielsweise durch abscheren, abreißen oder abbrechen eines vorstehenden Zapfens erreicht werden, der am Rückhalteelement 21 angeordnet ist.

Weiters ist es möglich, dass das Rückhalteelement in sich dermaßen durch ein deformierbares Material ausgebildet wird, sodass dieses bei Kontakt mit dem Anschlag 15 nachgiebt und unter diesem durchgleiten kann. Dadurch, dass sich aufgrund der Deformierung des Rückhaltelements 21 nun die Saugmechanik 8 in Bewegung setzen kann, da diese durch den Saugkraftspeicher 16 angetrieben wird, bewegt sich der Kolben 14 relativ zum Gehäuse 2 der Saugvorrichtung 3. Dabei erhöht sich das Volumen des Reservoirs 5 womit durch den entstehenden Unterdruck über die Nadel 4 die Flüssigkeitsprobe 60 aufgenommen wird.

Dadurch, dass die Vorschubbegrenzung 9 im Gehäuse 2 des Verstellabschnittes 20 zurückbleibt, ist ein Rückführen der Vorschubmechanik am Anschlag 15 und der Vorschubbegrenzung 9 nicht mehr möglich. Dies schließt eine Mehrfachverwendung der Vorrichtung 1 aus. Die Figur 6 zeigt den Saugbetriebszustand SB.

Vergleicht man die untereinanderliegenden Fig. 4 bis 6, erkennt man zwischen der Fig. 4 und der Fig. 5 den Vorschubbereich V, den die Nadel 4 oder die Saugvorrichtung 3 beim Betätigen des Auslösers 7 zurücklegt. Dies ist entsprechend über die Veränderung der Position der Vorschubbegrenzung 9 einstellbar. Vergleicht man die Fig. 5 mit der Fig. 6, erkennt man die Saugstrecke S, welche verantwortlich ist für das Volumen im Reservoir 5 und die Aufnahme der Flüssigkeitsprobe 60.

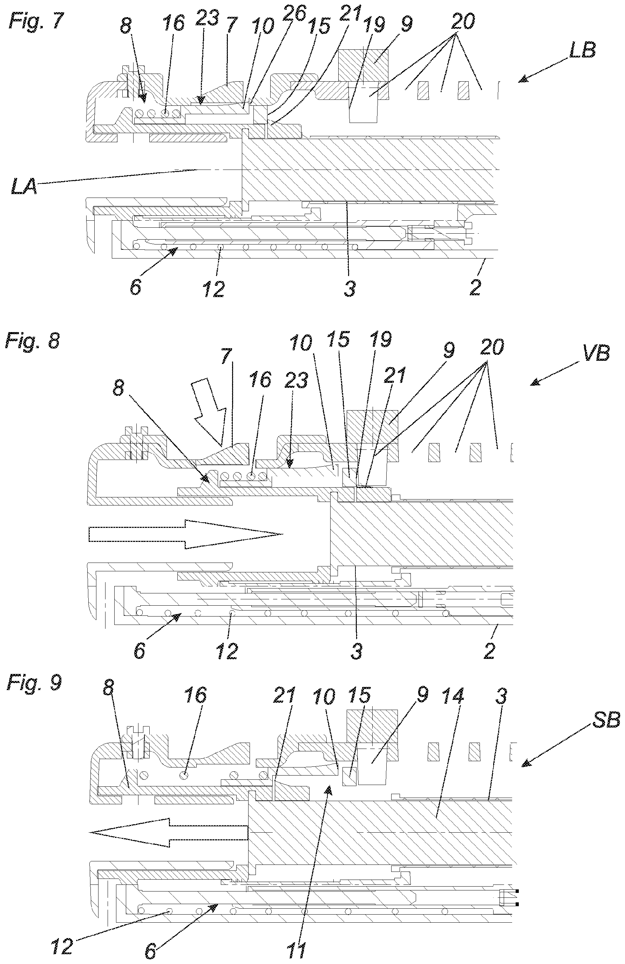

Die Fig. 7 zeigt detailliert, wie der Ladetriebszustand LB der Vorschubmechanik 6 und der Saugmechanik 8 realisiert wird. Der Saugkraftspeicher 16 ist in seiner geladenen Form. So auch der Vorschubkraftspeicher 12. Der Auslöser 7 kontaktiert das Arretiermittel 10, welches in Zusammenwirkung mit dem Anschlagelement 26 jedoch eine Bewegung der Vorschubmechanik 6 verhindert. Direkt neben dem rückfedernd ausgebildeten Arretiermittel 10 befindet sich der Anschlag 15, der bei der Bewegung der Vorschubmechanik 6 entlang der Längsachse LA mitbewegt wird.

Fig. 8 zeigt, wie sich im Vorschubsbetriebszustand VB der Vorschubkraftspeicher 12 entlädt. Das Arretiermittel 10 an der Kupplung 23 wurde dabei durch den Auslöser 7 in eine Auslösestellung gebracht, wodurch über den Vorschubkraftspeicher 12 die Vorschubmechanik 6 in Bewegung gesetzt worden ist und sich die Kupplung 23 mit dem Anschlag 15 gegen die korrespondierende Anschlagfläche 19 der Vorschubbegrenzung 9 bewegt.

Fig. 9 zeigt, wie sich ein Teil des Rückhaltelements 21 plastisch oder elastisch verformt hat, wodurch dieses den Weg für den Anschlag 15 entlang der Längsachse LA freigibt. Durch die Freigabe des Anschlags 15 kann sich nun die Saugmechanik 8 in Bewegung setzen, da diese durch den Saugfederspeicher 16 vorgespannt ist.

Die Blockiervorrichtung 11 zur Verhinderung einer Mehrfachverwendung wird somit durch ein Zusammenwirken mehrerer Bestandteile ausgebildet. Diese umfasst die Vorschubbegrenzung 9, den Anschlag 15 und das rückfedernde Arretiermittel 10.

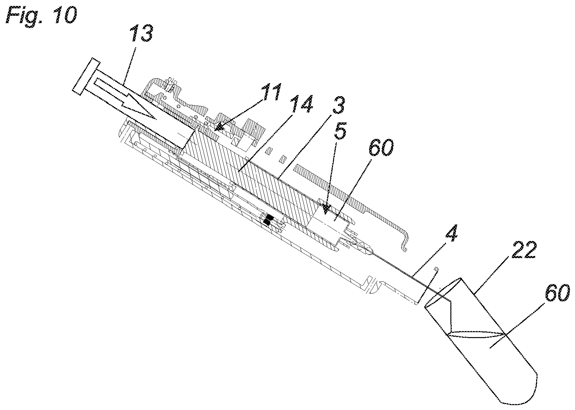

Fig. 10 zeigt, wie sich das Reservoir 5 durch die Beaufschlagung des Kolbens 14 leert. Die darin enthaltene Flüssigkeitsprobe 60 wird über die Nadel 4 oder auch direkt aus der Saugvorrichtung 3 in einen Probenbehälter 22 umgefüllt. Dies kann beispielsweise durch direktes Beaufschlagen des Kolbens 14 geschehen oder auch durch ein den Kolben kontaktierendes Hilfsmittel, wie beispielsweise die Spannvorrichtung 13. Die Blockiervorrichtung 11 verhindert, dass die Vorrichtung 1 nach der Durchführung einer Flüssigkeitsprobe erneut verwendet werden kann.

### Bezugszeichenliste

- 1: Vorrichtung
- 2: Gehäuse
- 3: Saugvorrichtung
- 4: Nadel
- 5: Reservoir
- 6: Vorschubmechanik
- 7: Auslöser
- 8: Saugmechanik
- 9: Vorschubbegrenzung
- 10: Arretiermittel
- 11: Blockiervorrichtung
- 12: Vorschubkraftspeicher
- 13: Spannvorrichtung
- 14: Kolben
- 15: Anschlag
- 16: Saugkraftspeicher
- 17: Haltevorrichtung
- 18: Kontaktabschnitt
- 19: Korrespondierende Anschlagfläche
- 20: Verstellabschnitt
- 21: Rückhaltelement
- 22: Probenbehälter
- 23: Kupplung
- 26: Anschlagelement
- 27: Führungselement
- 50: Körper
- 51: Bauchfell
- 52: Harnblase
- 53: Schambein
- 54: Bein
- 55: Bauchwand
- 56: Hohlraum
- 60: Flüssigkeitsprobe
- 61: Flüssigkeit
- LA: Längsachse
- LB: Ladebetriebszustand
- VB: Vorschubsbetriebszustand
- SB: Saugbetriebszustand

## Patentansprüche

1. Vorrichtung (1) zur Entnahme einer Flüssigkeitsprobe (60), insbesondere zur Durchführung einer suprapubischen Blasenpunktion, mit
- einem Gehäuse (2) und zumindest einer im oder am Gehäuse angeordneten Saugvorrichtung (3) und einem Reservoir (5) mit einem Volumen (V) für die Aufnahme der Flüssigkeitsprobe (60),
- einer Vorschubmechanik (6) zum Bewegen der Saugvorrichtung (3) relativ zum Gehäuse,
- einem Auslöser (7) zum Auslösen der Vorschubmechanik (6), wobei die Vorrichtung (1)
- in einem Ladebetriebszustand (LB) durch den Auslöser (7) in einen Vorschubsbetriebszustand (VB) bringbar ist, in dem sich die Saugvorrichtung (3) durch die Vorschubmechanik (6) relativ zum Gehäuse (2) bewegt, und anschließend
- selbsttätig in einen Saugbetriebszustand (SB) bringbar ist, in dem automatisch das Volumen (V) des Reservoirs (5) durch eine Saugmechanik (8) vergrößerbar ist,
**dadurch gekennzeichnet, dass** eine Blockiervorrichtung (11) vorgesehen ist, durch welche eine Mehrfachverwendung der Vorrichtung (1) verhinderbar ist.

2. Vorrichtung nach Anspruch 1, **dadurch gekennzeichnet, dass** die Vorrichtung (1) nach der Betätigung des Auslösers (7) durch zumindest einen, vorzugsweise durch eine Feder ausgebildeten und in oder an der Vorrichtung (1) angeordneten Kraftspeicher, automatisch vom Ladebetriebszustand (LB) in den Vorschubsbetriebszustand (VB) und darauffolgend automatisch in den Saugbetriebszustand (SB) wechselbar ist.

3. Vorrichtung nach Anspruch 1 oder 2, **dadurch gekennzeichnet, dass** die Vorschubmechanik (6) einen Vorschubkraftspeicher (12) aufweist, welcher beim Entspannen die Vorschubmechanik (6) im Vorschubbetriebszustand (VB) in Bewegung setzt und die Saugmechanik (8) einen Saugkraftspeicher (16) aufweist, welcher beim Entspannen die Saugmechanik (8) im Saugbetriebszustand (SB) in Bewegung setzt.

4. Vorrichtung nach einem der Ansprüche 1 bis 3, **dadurch gekennzeichnet, dass** die Saugvorrichtung (3) durch eine Injektionsspritze, vorzugsweise Einwegspritze, mit einer daran anordenbaren Nadel (4) ausgebildet ist.

5. Vorrichtung nach einem der Ansprüche 1 bis 4, **dadurch gekennzeichnet, dass** die Bewegung der Vorschubmechanik (6) relativ zum Gehäuse (2) durch eine Vorschubbegrenzung (9) einstellbar ist.

6. Vorrichtung nach einem der Ansprüche 3 bis 5, **dadurch gekennzeichnet, dass** der Auslöser (7) ein Arretiermittel (10) aufweist, wobei der Vorschubkraftspeicher (12) und/oder der Saugkraftspeicher (16) in einer vorgespannten Spannposition durch das Arretiermittel (10) feststellbar ist und das Arretiermittel (10) zum Entspannen des Vorschubkraftspeichers (12) und des Saugkraftspeichers (16) durch den Auslöser (7) lösbar ist.

7. Vorrichtung nach einem der Ansprüche 1 bis 6, **dadurch gekennzeichnet, dass** das Gehäuse (2) und die Saugvorrichtung (3) zumindest abschnittsweise durch ein transparentes Material ausgebildet sind, wodurch der Füllstand des Reservoirs (5) der Saugvorrichtung (3) und der Betriebszustand der Vorrichtung (1) erkennbar ist.

8. Vorrichtung nach einem der Ansprüche 3 bis 7, **dadurch gekennzeichnet, dass** eine Spannvorrichtung (13) vorgesehen ist, durch welche ein Spannen des Vorschubkraftspeichers (12) und/oder des Saugkraftspeichers (16) und somit das Schaffen des Ladebetriebszustandes (LB) durchführbar ist.

9. Vorrichtung nach Anspruch 8, **dadurch gekennzeichnet, dass** die Spannvorrichtung (13) zum Herausdrücken der Flüssigkeitsprobe (60) aus dem Reservoir (5) der Saugvorrichtung (3) verwendbar ist.

10. Vorrichtung nach einem der Ansprüche 3 bis 9, **dadurch gekennzeichnet, dass** sich der Vorschubkraftspeicher (12) im Ladebetriebszustand (LB) in einem gespannten Zustand und der Saugkraftspeicher (16) in einem entladenen Zustand befindet, wobei sich bei einer Entladung des im Vorschubbetriebszustand (VB) befindlichen Vorschubkraftspeichers (12) der Saugkraftspeicher (16) aufgrund einer zwischen der Vorschubmechanik (6) und der Saugmechanik (8) befindlichen Kupplung (23) in einen gespannten Zustand bewegt und anschließend selbsttätig im Saugbetriebszustand (SB) in den entspannten Zustand wechselbar ist, wobei das Volumen (V) des Reservoirs an der Saugvorrichtung (3) vergrößerbar ist.

## Claims

1. Device (1) for sampling a fluid sample (60), in particular for conducting a suprapubic bladder puncture, with
- a case (2) and at least one suction device (3) arranged at or in the case (2) and a reservoir (5) with a volume (V) for receiving the fluid sample (60),
- a feed mechanism (6) for moving the suction device (3) relative to the case,
- a trigger (7) for triggering the feed mechanism (6),
where the device (1)
- in a charge operating state (LB) can be brought into a feed operating state (VB) by means of the trigger (7), in which the suction device (3) moves relative to the case (2) by means of the feed mechanism (6), and subsequently
- can be automatically brought into a suction operating state (SB), in which the volume (V) of the reservoir (6) can be automatically enlarged by means of a suction mechanism (8),
**characterized in that** a blocking device (11) is provide, by means of which a multiple usage of the device (1) is preventable.

2. Device according to claim 1, **characterized in that** after actuation of the trigger (7) the device (1) is automatically switched from the charge operating state (LB) in the feed operating state (VB) and subsequently in the suction operating state (SB) by means of a force storage member, preferably a spring, which is arranged in or at the device (1).

3. Device according to claim 1 or 2, **characterized in that** the feed mechanism (6) comprises a feed force storage member (12), which on release moves the feed mechanism (6) in the feed operating state (VB) and the suction mechanism (8) comprises a suction force storage member (16), which on release moves the suction mechanism (8) in the suction operating state (SB).

4. Device according to one of the claims 1 to 3, **characterized in that** the suction device (3) is formed by an injection syringe, preferably a one-way syringe.

5. Device according to one of the claims 1 to 4, **characterized in that** the movement of the feed mechanism (6) relative to the case (2) can be adjusted by means of a feed limit (9).

6. Device according to one of the claims 3 to 5, **characterized in that** the trigger (7) comprises locking means (10), where the feed force storage member (12) and/or the suction force storage member (16) is lockable in a prestressed tensioned position by the locking means (10) and that the locking means (10) is releasable by the trigger (7) for releasing the feed force storage member (12) and the suction force storage member (16).

7. Device according to one of the claims 1 to 6, **characterized in that** the case (2) and the suction device (3) are at least partially formed by a transparent material, whereby a filling level of the reservoir (5) of the suction device (3) and the operating state of the device (1) is visible.

8. Device according to one of the claims 3 to 7, **characterized in that** a tensioning device (13) is provided, by means of which tensioning of the feed force storage member (12) and/or the suction force storage member (16) and thereby establishing of the charge operating state (LB) is achievable.

9. Device according to claim 8, **characterized in that** the tensioning device (13) is usable to push out the fluid sample (60) from the reservoir (5) of the suction device (3) .

10. Device according to one of the claims 3 to 9, **characterized in that** in a charge operating state (LB) the feed force storage member (12) is in a tensioned state and the suction force storage member (16) is in a released state, where on release of the feed force storage member (12), which is in the charge operating state (LB), the suction force storage member (16) is moved into a tensioned state by means of a clutch (23) arranged in between the feed mechanism (6) and the suction mechanism (8) and subsequently, in the suction operating state (SB), can be automatically brought into a released state, where the volume (V) of the reservoir at the suction device (3) can be enlarged.

## Revendications

1. Dispositif (1) pour prélever un échantillon de liquide (60), en particulier pour réaliser une ponction de vessie suspubienne, avec
- un boîtier (2) et au moins un dispositif d'aspiration (3) agencé dans ou au niveau du boîtier et un réservoir (5) avec un volume (V) pour la réception de l'échantillon de liquide (60),
- un mécanisme d'avance (6) pour le déplacement du dispositif d'aspiration (3) par rapport au boîtier,
- un déclencheur (7) pour le déclenchement du mécanisme d'avance (6),
dans lequel le dispositif (1)
- dans un mode de charge (LB) peut être amené par le déclencheur (7) dans un mode d'avance (VB), dans lequel le dispositif d'aspiration (3) se déplace par le mécanisme d'avance (6) par rapport au boîtier (2), et ensuite
- peut être amené automatiquement dans un mode d'aspiration (SB), dans lequel le volume (V) du réservoir (5) peut être augmenté automatiquement par un mécanisme d'aspiration (8),
**caractérisé en ce qu'**un dispositif de blocage (11) est prévu, par lequel une utilisation multiple du dispositif (1) peut être empêchée.

2. Dispositif selon la revendication 1, **caractérisé en ce que** le dispositif (1) peut passer après l'actionnement du déclencheur (7) par au moins un accumulateur de force agencé dans ou au niveau du dispositif (1) et réalisé de préférence par un ressort, automatiquement du mode de charge (LB) dans le mode d'avance (VB) et ensuite automatiquement dans le mode d'aspiration (SB).

3. Dispositif selon la revendication 1 ou 2, **caractérisé en ce que** le mécanisme d'avance (6) présente un accumulateur de force d'avance (12) qui met en mouvement lors de la détente le mécanisme d'avance (6) dans le mode d'avance (VB) et le mécanisme d'aspiration (8) présente un accumulateur de force d'aspiration (16) qui met en mouvement lors de la détente le mécanisme d'aspiration (8) dans le mode d'aspiration (SB).

4. Dispositif selon l'une quelconque des revendications 1 à 3, **caractérisé en ce que** le dispositif d'aspiration (3) est réalisé par une seringue d'injection, de préférence une seringue à usage unique, avec une aiguille (4) pouvant être agencée au niveau de celle-ci.

5. Dispositif selon l'une quelconque des revendications 1 à 4, **caractérisé en ce que** le mouvement du mécanisme d'avance (6) est réglable par rapport au boîtier (2) par une limitation d'avance (9).

6. Dispositif selon l'une quelconque des revendications 3 à 5, **caractérisé en ce que** le déclencheur (7) présente un moyen d'arrêt (10), dans lequel l'accumulateur de force d'avance (12) et/ou l'accumulateur de force d'aspiration (16) peut être bloqué dans une position de serrage précontrainte par le moyen d'arrêt (10) et le moyen d'arrêt (10) peut être détaché pour la détente de l'accumulateur de force d'avance (12) et de l'accumulateur de force d'aspiration (16) par le déclencheur (7).

7. Dispositif selon l'une quelconque des revendications 1 à 6, **caractérisé en ce que** le boîtier (2) et le dispositif d'aspiration (3) sont réalisés au moins par sections par un matériau transparent, par quoi le niveau de remplissage du réservoir (5) du dispositif d'aspiration (3) et le mode du dispositif (1) peuvent être détectés.

8. Dispositif selon l'une quelconque des revendications 3 à 7, **caractérisé en ce qu'**un dispositif de serrage (13) est prévu, par lequel un serrage de l'accumulateur de force d'avance (12) et/ou de l'accumulateur de force d'aspiration (16) et ainsi la création du mode de charge (LB) peuvent être réalisés.

9. Dispositif selon la revendication 8, **caractérisé en ce que** le dispositif de serrage (13) est utilisable pour extraire par pression l'échantillon de liquide (60) hors du réservoir (5) du dispositif d'aspiration (3).

10. Dispositif selon l'une quelconque des revendications 3 à 9, **caractérisé en ce que** l'accumulateur de force d'avance (12) dans le mode de charge (LB) se trouve dans un état serré et l'accumulateur de force d'aspiration (16) se trouve dans un état déchargé, dans lequel lors d'une décharge de l'accumulateur de force d'avance (12) se trouvant dans le mode d'avance (VB), l'accumulateur de force d'aspiration (16) se déplace en raison d'un couplage (23) se trouvant entre le mécanisme d'avance (6) et le mécanisme d'aspiration (8) dans un état serré et peut ensuite passer automatiquement dans le mode d'aspiration (SB) dans l'état desserré, dans lequel le volume (V) du réservoir peut être augmenté au niveau du dispositif d'aspiration (3).
